# EUROPEAN PATENT SPECIFICATION

(11) **EP 1 989 280 B1**
(45) Date of publication and mention of the grant of the patent: **19.05.2010**
(21) Application number: 06720613.6
(22) Date of filing: 10.02.2006
(51) Int. Cl.: C11D 1/62, C11D 3/00, C07C 219/06, A61K 8/41, A61Q 5/12

(54) **FABRIC CONDITIONING ACTIVE COMPOSITIONS**
TEXTILKONDITIONIERUNGSWIRKSTOFFE
COMPOSITIONS ACTIVES POUR LE CONDITIONNEMENT DE TISSUS

(43) Date of publication of application: 12.11.2008
(73) Proprietor: STEPAN COMPANY, Northfield, Illinois 60093 (US)
(72) Inventor: NEPRAS, Marshall J., Burlington, WI 53105 (US); TERRY, Michael R., Gurnee, IL 60031 (US); LEVINSON, Matthew I., Chicago, IL 60646 (US); BERNHARDT, Randal J., Antioch, IL 60002 (US)
(74) Representative: Schnappauf, Georg
(86) International application number: PCT/US2006/004746
(87) International publication number: WO 2007/092020

(56) References cited:
- EP-A- 0 835 863
- EP-A- 1 239 024
- WO-A-00/06678
- WO-A-01/32813

## Description

### Field of the Invention

The present invention relates to fabric or hair conditioning active compositions and methods for making them. More specifically, the invention relates to compositions comprising a mixture of esterquat compounds that exhibit improved rheological stability when formulated in a liquid composition, and methods for making them.

### Background of the Invention

Liquid fabric conditioning compositions that soften fabrics in the rinse cycle are known. Such compositions commonly comprise less than 7.5% by weight of conditioning active, in which case the composition is defined as "dilute," from 7.5% to about 30% by weight of conditioning active, in which case the compositions are defined as "concentrated," or more than about 30% by weight of conditioning active, in which case the composition is defined as "super-concentrated."

Concentrated and super-concentrated compositions are desirable since these require less packaging and are therefore environmentally more compatible than dilute or semi-dilute compositions.

A problem frequently associated with fabric conditioning compositions, as defined above, is that the product is not stable upon storage, especially when stored at high temperatures. Instability can manifest itself as a thickening of the product upon storage, even to the point that the product is no longer pourable.

The problem of thickening upon storage is particularly apparent in concentrated and super concentrated fabric conditioning compositions comprising esterquat fabric conditioning material having one or more fully saturated alkyl chains.

However, it is desirable to use esterquat compounds due to their inherent biodegradability. It is also desirable to use substantially fully saturated esterquat fabric conditioning compounds due to their excellent conditioning capabilities, and because they are more stable to oxidative degradation (which can lead to malodour generation) than partially saturated or fully unsaturated esterquat conditioning compounds.

Of the types of esterquat materials known, it is desirable to use those based on triethanolamine which comprise at least some mono-esterquat component and at least some tri-esterquat component

A further problem known to affect concentrated and super-concentrated fabric conditioning compositions is that the initial viscosity of a fully formulated composition can be so high that the composition is substantially unpourable.

The problem of undesirably high initial viscosity and poor viscostability (stability of viscosity over time) upon storage has previously been addressed in various ways.

It has now been found that the viscostability of a fabric conditioning composition can be controlled by a particular selection of the ester distribution and by maintaining low levels of ester amine and free fatty acid.

### Summary of the Invention

The present invention provides a fabric conditioner active composition comprising a mixture of quaternised mono-, di-, and tri-esters of alkanolamines (an "esterquat mixture") in which the tri-esterquat content is from 25 to 50% by weight of said esterquat mixture. When the tri-esterquat content is from 25 to 30% by weight of said esterquat mixture the free amine content of the composition is less than 0.5% by weight of said esterquat mixture. When the tri-esterquat content is more than 30% by weight of said esterquat mixture the free amine content is less than 6% by weight of said esterquat mixture. Finally, the composition contains less than 1% by weight of free fatty acid based on the weight of said esterquat mixture.

### Detailed Description of the Invention

The fabric conditioning active material of the present invention is useful, for example, in formulating fabric conditioning and antistatic compositions used, for example, in a laundering process.

### Esterquat fabric conditioning active material

The fabric conditioning active material of the present invention comprises an esterquat mixture of quaternised mono-ester, di-ester, and tri-ester components wherein the tri-esterquat component is from 25 to 50% by weight of the esterquat mixture. (The sum of the weights of mono-esterquat, di-esterquat, and tri-esterquat is 100% by weight of the esterquat mixture.)

By mono-, di- and tri-esterquat components, it is meant that the esterquat conditioning material comprises, respectively, an mono-esterquat (a esterquat compound comprising a single ester link with a fatty hydrocarbyl chain attached thereto), an di-esterquat (a esterquat compound comprising two ester links each of which has a fatty hydrocarbyl chain attached thereto), and an tri-esterquat (a esterquat compound comprising three ester links each of which has a fatty hydrocarbyl chain attached thereto).

In this specification, a "fatty hydrocarbyl chain," such as "R" in the amine and esterquat structures in this specification, is the alkyl or alkenyl moiety of a fatty acid, having one less carbon atom than the corresponding complete fatty acid. (The final carbon atom of the complete fatty acid is the acyl carbon of the ester linkage.) For example, stearic acid has 1 carbon atoms, arranged in the molecular formula:

CH₃(CH₂)₁₆COOH

The corresponding fatty hydrocarbyl chain is CH₃(CH₂)₁₆-, alternatively referred to here as a C₁₇ alkyl moiety. If unsaturated (for example, the fatty hydrocarbyl chain of oleic acid - a monounsaturate), it would be a C₁₇ alkenyl moiety.

Preferably, the average chain length of the alkyl or alkenyl moiety is at least C₁₃, alternatively at least C₁₅. In one contemplated embodiment, at least half of the chains have a length of C₁₇.

It is contemplated in one embodiment that the alkyl or alkenyl chains can be predominantly linear.

The fatty acid hydrocarbyl chain can be contributed by various starting materials, for example free fatty acids, either separately or in combinations, such as fatty acid mixtures characteristic of the fatty acid constituents of glyceride esters in natural tallow, palm kernel oil, or other suitable feedstocks. Other contemplated starting materials are glyceride mono-, di-, or tri-esters of fatty acids (for example, tallow or palm kernel oil), lower alkyl esters of fatty acids (for example methyl esters), acid chlorides corresponding to fatty acids, and other compounds, as is well known in the art. Again, these fatty acid derivatives may be made from a single fatty acid or mixtures of fatty acids, such as those derived from natural fatty acid feedstocks.

The contemplated esterquat cationic conditioning material for use in the invention is represented by formula (I):

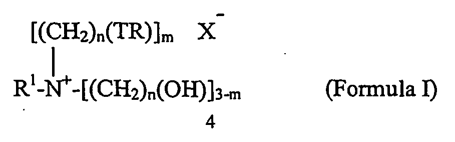

Each R is independently selected from a C₅₋₃₅ alkyl or alkenyl group, optionally a C₇₋₂₁ alkyl or alkenyl group, optionally a C₁₁₋₂₁ alkyl or alkenyl group, optionally an at least predominantly C₁₃₋₁₇ alkyl or alkenyl group. R¹ represents a C₁₋₄ alkyl or hydroxyalkyl group or a C₂₋₄ alkenyl group,
T is 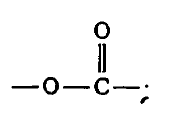 n is an integer selected from 1 to 4, optionally selected from 2 to 4; m is 1 for an mono-esterquat, 2 for an di-esterquat, or 3 for an tri-esterquat, and denotes the number of moieties to which it refers that pend directly from the N atom, and X⁻ is an anionic group, such as a halide or alkyl sulphate, for example, a C₁₋₄ alkyl or hydroxyalkyl sulfate or a C₂₋₄ alkenyl sulfate. Specifically contemplated anionic groups include chloride, methyl sulphate or ethyl sulphate.

Contemplated materials within this class are esters of triethanolammonium methyl sulphate, particularly tallow or hardened tallow esters.

The tri-esterquat content of the fabric conditioning material is from 25 to 50% by weight This tri-esterquat content can be provided in a variety of ways, as by using a relatively high ratio of the fatty acid to the starting alkanolamine in the reaction mixture, using an optimal amount of a suitable catalyst in the reaction mixture for promoting triester formation, raising the temperature of the reaction mixture relatively slowly, and other expedients known to the skilled person.

The contemplated di-esterquat content of the esterquat mixture is at most 70%, optionally at most 60%, optionally at most 50%, optionally at most 40% optionally at most 30% by weight of the esterquat mixture. The contemplated di-esterquat content of the esterquat mixture is at least 30%, optionally at least 40%, optionally at least 50%, optionally at most 55%, optionally at least 60%, optionally at least 65% by weight of the esterquat mixture.

The weight percentages of the mono-, di-, and tri-esterquats in the esterquat mixture are reported on the basis of the total weight of the three. Thus, the sum of these three percentages is 100%. The weight percentages of free amine and fatty acid in the esterquat mixture are also stated here based on the total weight of mono-, di-, and tri-esterquats in the esterquat mixture.

In practice it has been found that conditioner compositions having higher tri-esterquat contents can tolerate more free amine content in the composition without deleteriously affecting viscostability. When the tri-esterquat content is from 25 to 30% by weight the free amine content of the composition is contemplated to ideally be low, i.e below 0.5% by weight When the tri-esterquat content is greater than 30% by weight additional free amine may be tolerated in the composition, and the free amine conten, is less than 6% by weight of the esterquat mixture.

The free (i.e. unquaternized) amine content may be adjusted by controlling the reaction conditions of the preparation of the esterquats, for example, (1) by charging a suitable amount of quaternising agent close to a 1:1 molar ratio of amines to quaternizing agent, (2) by carrying out the reaction long enough to consume the desired percentage of free amine present at the beginning of the quaternisation reaction, or (3) by selecting a suitable quaternising agent (for example, dimethyl sulphate is contemplated). Alternatively and/or in addition, an acid may be added toward the end of the preparation of the esterquats and/or during the preparation of the fabric conditioning composition to reduce the free amine content

The compositions contain less than 1% by weight of free fatty acid based on the exterquat mixture. A low free fatty acid content can be provided, for example, by reacting a fatty acid or a parent fatty acyl compound (such as a glyceride, alkyl ester, or acid chloride) and trialkanolamine under conditions, such as a low enough ratio of fatty acid to trialkanolamine and a long enough reaction time at elevated temperature, effective to consume at least 99% of the initial charge of fatty acid or parent

### Iodine Value of the Parent Fatty Acyl group or Acid

The iodine value of the parent fatty acyl compound or acid from which the esterquat fabric conditioning material is formed is from 0 to 20, preferably from 0 to 5, alternatively from 0 to 2. Alternatively the iodine value of the fatty acid or parent fatty acyl group from which the esterquat fabric conditioning material is formed is from 0 to 1. That is, it is contemplated that the alkyl or alkenyl chains are fully or substantially fully saturated.

If there is any unsaturated esterquat fabric conditioning material present in the composition, the iodine value, referred to above, represents the mean iodine value of the parent fatty acyl compounds or fatty acids of all of the esterquat materials present

In the context of the present invention, the iodine value of the parent fatty acyl compound or acid, from which the fabric conditioning material is formed, is defined as the number of grams of iodine which react with 100 grams of the parent compound.

In the context of the present invention, the method for calculating the iodine value of a parent fatty acyl compound/acid comprises dissolving a prescribed amount (from 0.1-3g) into about 15ml chloroform. The dissolved parent fatty acyl compound/fatty acid is then reacted with 25 ml of iodine monochloride in acetic acid solution (0.1M). To this, 20ml of 10% potassium iodide solution and about 150 ml deionised water are added. After addition of the halogen has taken place, the excess of iodine monochloride is determined by titration with sodium thiosulphate solution (0.1M) in the presence of a blue starch indicator powder. At the same time a blank is determined with the same quantity of reagents and under the same conditions. The difference between the volume of sodium thiosulphate used in the blank and that used in the reaction with the parent fatty acyl compound or fatty acid enables the iodine value to be calculated.

The esterquat fabric conditioning material of formula (I) is present in an amount from 1 to 80% by weight of esterquat material (active ingredient) based on the total weight of the composition, generally 2 to 60% by weight, for example 5 to 25% by weight.

Broadly speaking, the conditioning active compositions of the present invention, also known as esterquats, are made by combining a fatty acid source and an alkanolamine, typically at a starting temperature at which the fatty acid source is molten, optionally adding a catalyst, then heating the reaction mixture while drawing vacuum until the desired endpoint(s), such as acid value and final alkalinity value, are reached. The resulting esteramine intermediate is then quaternised using an alkylating agent, yielding an esterquat product. The esterquat product is a mixture of quaternised monoester, diester, and triester components and optionally some amount of one or more reactants, intermediates, and byproducts, including but not limited to free amine and free fatty acid or parent fatty acyl compounds.

The fabric conditioning active compositions of the present invention can be formulated with water and other ingredients to provide rinse conditioner compositions for use in the rinse cycle of a domestic or commercial laundry process. Some examples of contemplated other ingredients follow.

### Fatty Complexing Agent

The rinse conditioner compositions may comprise a fatty complexing agent. Especially suitable fatty complexing agents include fatty alcohols.

Contemplated fatty alcohols include hardened tallow or predominantly C₁₆-C₁₈ alcohols (available under the trade names Stenol® and Hydrenol® (obtainable from Cognis Deutschland GmbH & Co., Duesseldorf, Germany), and Laurex® CS, obtainable from Laurex Group, Inc., Plano, Texas USA) and behenyl alcohol, a C₂₂ chain alcohol, available as Lanette® 22 (obtainable from Cognis Deutschland GmbH & Co., Duesseldorf, Germany).

The fatty complexing agent is present in a minimum amount greater than 0.2%, alternatively in an amount greater than 0.25%, alternatively in an amount greater than 0.3%, alternatively in an amount greater than 1.5%, alternatively in an amount of at least 1.6%, alternatively in an amount of at least 1.7%, alternatively in an amount of at least 1.8%, based on the total weight of the composition. The fatty complexing agent is present in a maximum amount of 15%, alternatively 10%, alternatively 5%, alternatively 4% by weight, on the same basis.

The inventors specifically contemplate the combination of any minimum value and any maximum value stated above to form a range that is also specifically contemplated herein. Some specific ranges contemplated here include an amount of from greater than 0.2% to 15%, alternatively from 0.25 to 5%, alternatively from 0.3 to 1% by weight. All percentages and proportions in this specification are by weight unless otherwise indicated.

### Nonionic surfactant

It is contemplated that the rinse conditioner compositions can optionally comprise a nonionic surfactant, though in some embodiments of the invention it is contemplated that a nonionic surfactant will not be present. A nonionic surfactant can be included as desired or needed for the purpose of stabilising the compositions.

Suitable nonionic surfactants include addition products of ethylene oxide and/or propylene oxide with fatty alcohols.

Any of the alkoxylated materials of the particular type described hereinafter can be used as the nonionic surfactant.

Suitable surfactants are substantially water-soluble surfactants of the general formula:

R-Y-(C₂H₄O)_{z}-C₂H₄OH

where R is selected from the group consisting of primary, secondary and branched chain alkyl and/or acyl hydrocarbyl groups; primary, secondary and branched chain alkenyl hydrocarbyl groups; and primary, secondary and branched chain alkenyl-substituted phenolic hydrocarbyl groups; the hydrocarbyl groups having a chain length of from 8 to about 25, preferably 10 to 20, for example 14 to 18 carbon atoms.

In the general formula for the ethoxylated nonionic surfactant, Y is typically: 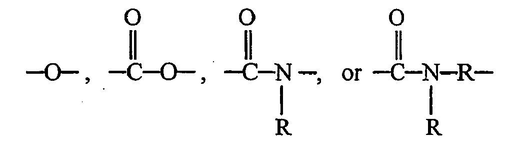 in which R has the meaning given above or can be hydrogen; and Z is at least about 8, preferably at least about 10 or 11.

Preferably the nonionic surfactant has an HLB of from about 7 to about 20, alternatively from 10 to 18, alternatively from 12 to 16.

Examples of nonionic surfactants follow. In the examples, the integer X in "EO(X)" defines the number of ethoxy (EO) groups in the molecule.

### A. Straight-Chain, Primary Alcohol Alkoxylates

The deca-, undeca-, dodeca-, tetradeca-, and pentadecaethoxylates (i.e. X is 10, 11, 12, 14, or 15, respectively) of n-hexadecanol, and n-octadecanol, having an HLB within the range recited herein, are useful viscosity/dispersibility modifiers in the context of this invention. Exemplary ethoxylated primary alcohols useful herein as the viscosity/dispersibility modifiers of the compositions are C₁₈ EO(10) and C₁₈ EO(11). The ethoxylates of mixed natural or synthetic alcohols in the "tallow" or "coco"chain length ranges are also useful herein. Specific examples of such materials include tallow alcohol-EO(11), tallow alcohol-EO(18), and tallow alcohol-EQ (25), coco alcohol-EO(10), coco alcohol-EO(15), coco alcohol-EO(20) and coco alcohol-EO(25).

### B. Straight-Chain, Secondary Alcohol Alkoxylates

The deca-, undeca-, dodeca-, tetradeca-, pentadeca-, octadeca-, and nonadeca-ethoxylates (i.e. X is 10, 11, 12, 14, 15, 18, or 19, respectively) of C₁₆ to C₂₀ secondary alcohols such as 3-hexadecanol, 2-octadecanol, 4-eicosanol, and 5-eicosanol having an HLB within the range recited herein are useful viscosity and/or dispersibility modifiers in the context of this invention. Exemplary ethoxylated secondary alcohols useful herein as the viscosity and/or dispersibility modifiers of the compositions are: C₁₆ EO(11); C₂₀ EO(11); and C₁₆
EO(14).

### C. Alkyl Phenol Alkoxylates

As in the case of the alcohol alkoxylates, the hexa- to octadeca-ethoxylates (i.e. X is 6 to 18) of alkylated phenols, particularly monohydric alkylphenols, having alkyl chain lengths of 12 to 16 carbon atoms and an HLB within the range recited herein are useful as the viscosity and/or dispersibility modifiers of the instant compositions. The hexa- to octadeca-ethoxylates of p-tridecylphenol, m-pentadecylphenol, and the like, are useful herein. Exemplary ethoxylated alkylphenols useful as the viscosity and/or dispersibility modifiers of the esterquat mixtures herein are: p-tridecylphenol EO(11) and p-pentadecylphenol EO(18).

As used herein and as generally recognized in the art, a phenylene group in the nonionic formula is the equivalent of an alkylene group containing from 2 to 4 carbon atoms. For present purposes, nonionics containing a phenylene group are considered to contain an equivalent number of alkyl carbon atoms calculated as the sum of the carbon atoms in the alkyl group plus about 3.3 carbon atoms for each phenylene group.

### D. Olefinic Alkoxylates

The alkenyl alcohols, both primary and secondary, and alkenyl phenols described above can be ethoxylated to an HLB within the range recited herein and used as the viscosity and/or dispersibility modifiers of the instant compositions.

### E. Branched Chain Alkoxylates

Branched chain primary and secondary alcohols, which are available from the well-known "OXO" process, can be ethoxylated and employed as the viscosity and/or dispersibility modifiers of compositions herein.

### F. Polyol Based Surfactants

Suitable polyol based surfactants include sucrose esters such sucrose monooleates, alkyl polyglucosides such as stearyl monoglucosides and stearyl triglucoside and alkyl polyglycerols.

The above nonionic surfactants are useful in the present compositions alone or in combination, and the term "nonionic surfactant" encompasses mixed nonionic surface-active agents.

The nonionic surfactant is optional, and if used can be present in an amount from 0.01 to 10%, alternatively 0.1 to 5%, alternatively 0.35 to 3.5%, alternatively 0.5 to 2% by weight, based on the total weight of the rinse conditioner composition.

### Perfume

The rinse conditioner compositions can optionally further comprise one or more perfumes:

The hydrophobicity of the perfume and oily perfume carrier are measured by *C log P. C log P* is calculated using the *"C log P"* program (calculation of hydrophobicities as log P (oil/water)) version 4.01, available from Daylight Chemical Information Systems Inc of Irvine California, USA.

It is well known that perfume is provided as a mixture of various components.

It is contemplated that at least a quarter (by weight) or more, preferably a half or more of the perfume components have a *C log P* of 2.0 or more, alternatively 3.0 or more, alternatively 4.5 or more, alternatively 10 or more.

Suitable perfumes having a *C log P* of three or more are disclosed in US 5500137.

The perfume can optionally be present in an amount from 0.01 to 10% by weight, alternatively 0.05 to 5% by weight, alternatively 0.5 to 4.0% by weight, based on the total weight of the composition.

### Liquid Carrier

The rinse conditioner compositions can optionally further comprise a liquid carrier. The liquid carrier employed in the instant compositions is preferably water due to its low cost relative availability, safety, and environmental compatibility. The level of water in the liquid carrier is more than about 50%, optionally more than about 80%, alternatively more than about 85%, by weight of the carrier. The level of liquid carrier is greater than about 50%, optionally greater than about 65%, alternatively greater than about 70%. Mixtures of water and a low molecular weight, for example <100, organic solvent, for example a lower alcohol such as ethanol, propanol, isopropanol or butanol are useful as the carrier liquid. Low molecular weight alcohols including monohydric, dihydric (glycol, etc.) trihydric (glycerol, etc.), and polyhydric (polyols) alcohols are also suitable carriers for use in the compositions of the present invention.

### Co-active conditioners

The rinse conditioner compositions can optionally further comprise co-active conditioners. These co-active conditioners may also be incorporated in an amount from 0.01 to 20% by weight, alternatively 0.05 to 10%, based on the total weight of the rinse conditioner composition. Contemplated co-active conditioners include fatty esters, and fatty N-oxides.

Contemplated fatty esters include fatty monoesters, such as glycerol monostearate (GMS). If GMS is present, then it is contemplated that the level of GMS in the composition can be from 0.01 to 10 wt.%, based on the total weight of the composition.

The co-active conditioner may also comprise an oily sugar derivative. Suitable oily sugar derivatives, their methods of manufacture and their contemplated amounts are described in WO O1/46361 on page 5 line 16 to page 11 line 20, the disclosure of which is incorporated herein.

### Viscosity control agents

The rinse conditioner compositions can optionally further comprise one or more polymeric viscosity control agents. Suitable polymeric viscosity control agents include nonionic and cationic polymers, such as hydrophobically modified cellulose ethers (for example Natrosol® Plus, obtainable from The Aqualon Division of Hercules, Inc., Wilmington, Delaware USA), cationically modified starches (for example Softgel^{™} BDA and Softgel^{™} BD, both obtainable from Avebe BA Corp., Veendam, Netherlands). A particularly contemplated viscosity control agent is a copolymer of methacrylate and cationic acrylamide available under the trade name Flosoft^{™} 200 (obtainable from SNF Floerger, Andrezieux, France).

Viscosity control agents are optionally present in an amount of 0.01 to 5 wt.%, alternatively 0.02 to 4 wt.%, based on the total weight of the composition.

### Further Optional Ingredients

The rinse conditioner compositions can optionally further comprise other optional nonionic conditioners, bactericides, soil-release agents, and other ingredients.

The compositions may also contain one or more optional ingredients conventionally included in fabric conditioning compositions such as pH buffering agents, perfume carriers, fluorescers, colourants, hydrotropes, antifoaming agents, antiredeposition agents, polyelectrolytes, enzymes, optical brightening agents, anti-shrinldng agents, anti-wrinkle agents, anti-spotting agents, antioxidants, sunscreens, anti-corrosion agents, drape imparting agents, anti-static agents, ironing aids, dyes etc.

### Product Form

At ambient temperature the fabric conditioner active composition can comprise an aqueous liquid or a solution or dispersion of the active agent in a solvent or dispersing medium, although neat liquid or solid compositions not containing a solvent are also contemplated. Aqueous dispersions of the esterquat conditioning material are particularly contemplated.

### Product Use

The composition can be used, for example, in the rinse cycle of a home washing machine, where it may be added directly in an undiluted state, for example through a dispenser drawer or, for a top-loading washing machine, directly into the drum. Alternatively, it can be diluted prior to use. The compositions may also be used in hand washing or in a domestic or commercial automatic laundry operation.

The compositions of the present invention can also be used in industrial fabric treatment operations; for example as a finishing agent for conditioning new clothes prior to sale to consumers.

The compositions are not limited to fabric conditioning uses, and may be formulated for use as hair conditioners or other products.

### Preparation

The compositions of the invention may be prepared according to any suitable method.

In a first contemplated method, the esterquat mixture, fatty complexing agent, nonionic stabilising agent and perfume are heated together until a co-melt is formed. Water is then heated and the co-melt is added to water with stirring. The formulation is then allowed to cool. In an alternative method, the perfume can be added to the formulation after the co-melt is formed, for example at any time during the cooling stage. Further embodiments can be found in the dependent claims.

### Examples

The invention will now be illustrated by the following non-limiting examples. Further modifications will be apparent to the person skilled in the art.

In the present specification, numbers represents examples of the invention. Letters represents comparative samples. All values are % by weight of the active ingredient unless stated otherwise.

### Example A (Comparative)

Tests are conducted using a esterquat fabric conditioning agent which is a esterquat mixture of hardened tallow esters of triethanolamine, quatemised with methyl sulphate, having a monoester/diester/triester ratio of 20%/60%/20% by weight (Quat). The material also comprises 8 to 10% by weight of esteramine, about 1% by weight of fatty acid and 15% isopropyl alcohol as solvent [N.B. all subsequent raw material examples contain 15% IPA].

Fabric conditioning compositions are prepared comprising the above Quat and fatty alcohol, with weight ratios of Quat to fatty alcohol of 12 : 1, 9 : 1 and 8 : 1 and with a fixed concentration of 5% by weight Quat (excluding IPA) in water (see Table 1).

The composition additionally comprises 05% of Natrosol Plus 331 (a hydrophobically modified hydroxyethyl cellulose obtainable from The Aqualon Division of Hercules, Inc., Wilmington, Delaware USA).

**Table 1**

| | Sample A1 | Sample A2 | Sample A3 |
|---|---|---|---|
| Quat | 5.88 | 5.88 | 5.88 |
| Fatty Alcohol¹ | 0.41 | 0.55 | 0.62 |
| Perfume | 0.32 | 0.32 | 0.32 |
| Polymer² | 0.05 | 0.05 | 0.05 |
| Minors (dye, preservative, antifoam) | | | |
| Water | to 100% | to 100% | to 100% |

| | | | |
|---|---|---|---|
| ¹ Stenol 1618L (obtainable from Cognis Deutschland GmbH & Co., Duesseldorf, Germany) ² Natrasol Plus 331 (obtainable from The Aqualon Division of Hercules, Inc., Wilmington, Delaware USA) | | | |

The compositions are prepared by adding the molten active blend (Quat plus Stenol®) to hot water 65°C with stirring. Once all the active is added the esterquat mixture is allowed to cool to 40°C with stirring at which point the perfume is added. The composition is further cooled to 30°C at which point the polymer is added as a 1% solution in water. The resulting formulations are stored at 45°C and the viscosity is measured periodically. The results are registered in Table 2. The figures in the table represent viscosity measurements (in units of mPa.s) as measured on a Haake RS600 Viscometer at a shear rate of 106 s⁻¹.

**Table 2**

| Example | Quat: fatty-OH | 0 | 2 weeks | 4 weeks | 6 weeks | 8 weeks | 12 weeks |
|---|---|---|---|---|---|---|---|
| A1 | 12:1 | 116 | 137 | 126 | 100 | 152 | 436 |
| A2 | 9:1 | 155 | - | 120 | 75 | 160 | 600 |
| A3 | 8:1 | 125 | 167 | 135 | 93 | 180 | 470 |

The formulations exhibit a significant increase in thickening after eight weeks. The primary cause of the instability is believed to be the hydrolysis of the esterquat, which produces insoluble fatty acid.

### Example B & C (Comparative)

Samples are prepared to assess the effect of additional esteramine in the raw material fabric composition. The compositional data of the esterquat raw material used as fabric conditioner in each sample is reported in Table 3 and the formulation details are reported in Table 4.

**Table 3**

| Component | Sample B | Sample C |
|---|---|---|
| MEQ/rel.wt.% | 17.8 | 17.8 |
| DEQ/rel.wt.% | 55.8 | 56.4 |
| TEQ/rel.wt.% | 26.4 | 25.8 |
| Esteramine/wt.% | 7.79 | 14.43 |
| Free fatty acid/wt.% | 0.68 | 0.76 |

| | | |
|---|---|---|
| Whereby MEQ = monoester quat, DEQ = diester quat and TEQ = triester quat | | |

**Table 4**

| | Sample B | Sample C |
|---|---|---|
| Esterquat raw material | 5.53 | 5.53 |
| Fatty Alcohol ¹ | 0.39 | 0.39 |
| Perfume | 0.34 | 0.34 |
| Minors (dye, preservative, antifoam) | | |
| Water | to 100% | to 100% |

| | | |
|---|---|---|
| ¹ Stenol® 1618L (ex. Cognis) | | |

The samples are prepared by adding the molten active premix to water at 70°C with stirring. The hot mixture is then mixed for a further 10 minutes at 70°C before being cooled to 40°C at which point perfume is added. After perfume addition, the esterquat mixture is cooled to room temperature prior to discharge.

The formulations are stored at 45°C and the viscosity measured periodically. The results are reported in Table 5. The figures in the table represent viscosity measurements (in units of mPa.s) as measured on a Haake RS600 Viscometer at a shear rate of 106 s⁻¹.

**Table 5**

| Sample | 0 weeks | 2 weeks | 4 weeks | 6 weeks | 8 weeks |
|---|---|---|---|---|---|
| Example B | 68 | 68 | 85 | 56 | 418 |
| Example C | 53 | 56 | 57 | 300 | 950 |

The results reveal that increasing the esteramine content in the composition provokes faster thickening at elevated temperature.

The formulation composition data for the fabric conditioner in the samples after 8 weeks storage at 45°C is measured. The percentage of free fatty acid based on the total weight of esterquat and fatty acid is reported in Table 6.

**Table 6**

| Component | Sample B | Sample C |
|---|---|---|
| MEQ/wt.% | 1.86 | 1.2 |
| DEQ/wt.% | 1.09 | 0.58 |
| TEQ/wt.% | 0.09 | 0.06 |
| Total esterquat/wt.% | 3.04 | 1.84 |
| Free fatty acid/wt.% | 1.73 | 1.54 |
| Wt % fatty, acid of total solids | 36.3 | 45.5 |

The resultant compositional data confirms that the faster increase in viscosity for Sample C correlates with a faster rate of hydrolysis for the sample containing the higher level of amine as the wt. % fatty acid in Sample C is significantly higher than it is for Sample B.

### Example D & E (Comparative)

Additional investigations on the effect of amine are conducted by adding extra esteramine during the making of the fabric conditioner formulation. The esterquat active used is the same esterquat raw material that was used for Example A.

The formulation details in weight percent are reported in Table 7. The compositions are prepared by adding the molten actives (easterquat, fatty alcohol, nonionic and ester amine where relevant) to water at 65°C. After the active addition, the resultant esterquat mixture is mixed using a high shear mixer and the composition is then cooled to 45°C, at which point the perfume is added. The product is allowed to cool to 30°C prior to discharge.

**Table 7**

| Component | Sample D | Sample E |
|---|---|---|
| Esterquat raw material | 16.44 | 14.95 |
| Fatty alcohol¹ | 1.1 | 1.1 |
| Nonionic² | 0.42 | 0.42 |
| Additional esteramine | 0 | 1.26 |
| Perfume | 0.6 | 0.6 |
| Water | balance | balance |

| | | |
|---|---|---|
| ¹ Stenol 1618L (ex. Cognis) ² Genapol C200 (obtainable from Clariant) | | |

The compositional data for the formulations after 12 months at room temperature (RT) storage is reported in Table 8.

**Table 8**

| | Total wt.% esteramine in formulation at time zero | MEQ Wt.% after 12 months at RT | DEQ Wt.% after 12 months at RT | TEQ Wt.% after 12 months at RT | Total esterquat Wt.% after 12 months at RT |
|---|---|---|---|---|---|
| Example D | 1.35 | 4.05 | 2.05 | 0.13 | 6.23 |
| Example E | 2.61 | 1.79 | | 0.04 | 2.48 |

The data shows the presence of esteramine increases hydrolysis of the esterquat.

### Examples F, G & H (Comparative)

Investigations are conducted to determine the effect of the free fatty acid on viscostability. The formulations reported in Table 9 are prepared in which all figures are weight percentages. The esterquat active used is the same esterquat raw material that is used for Example A. As detailed in example A, the esteramine content in the esterquat is in the range 8 to 10% by weight The process used is the same as that for Examples B and C.

**Table 9**

| Component | Example F | Example G | Example H |
|---|---|---|---|
| Esterquat raw material | 5.53 | 5.53 | 5.53 |
| Fatty alcohol¹ | 0.2 | 0.2 | 0 |
| Fatty acid² | 0 | 0.2 | 0.4 |
| Perfume | 0.34 | 0.34 | 0.34 |
| Water | balance | balance | balance |

| | | | |
|---|---|---|---|
| ¹ Stenol 1618L (obtainable from Cognis) ² Pristerine 4916 (obtainable from Uniquema) | | | |

The formulations are stored at 45°C and the viscosity measured periodically. The results are reported in Table 10. The figures in the table represent viscosity measurements (in units of mPa.s) as measured on a Haake RS600 Viscometer at a shear rate of 106 s⁻¹.

**Table 10**

| | 0 weeks | 2 weeks | 4 weeks | 6 weeks | 8 weeks | 12 weeks |
|---|---|---|---|---|---|---|
| Example F | 17 | 30 | 26 | 19 | 38 | 277 |
| Example G | 70 | 75 | 65 | gel | - | - |
| Example H | 70 | 40 | 166 | gel | - | - |

Samples G and H containing free fatty acid gel after 6 weeks demonstrating the negative impact that fatty acid has on the stability of the product.

### Examples I, J & K (Comparative) and Example 1

Investigations are conducted to determine the effect of esterquat distribution on viscostability. The compositional data for the esterquat raw material of the fabric conditioner is reported in Table 11.

**Table 11**

| | Example I | Example J | Example K | Example 1 |
|---|---|---|---|---|
| MEQ/rel.wt.% | 18.9 | 17.7 | 17.0 | 15.3 |
| DEQ/rel.wt.% | 59.4 | 57.4 | 56.2 | 54.8 |
| TEQ/rel.wt.% | 21.7 | 25.0 | 26.8 | 29.8 |
| Esteramine/wt.% | 8.51 | 5.76 | 3.10 | 0.27 |
| Free fatty acid/wt.% | 0.3 | 0.4 | 0.4 | 0.6 |

The formulations are detailed in Table 12. The process used to make the samples is the same as in Examples B and C.

**Table 12**

| | Example I | Example J | Example K | Example 1 |
|---|---|---|---|---|
| Esterquat raw material | 5.53 | 5.53 | 5.53 | 5.53 |
| Fatty Alcohol ¹ | 0.39 | 0.39 | 0.39 | 0.39 |
| Perfume | 0.34 | 0.34 | 0.34 | 0.34 |
| Minors (dye, preservative, antifoam) | Amount not reported | | | |
| Water | to 100% | to 100% | to 100% | to 100% |

| | | | | |
|---|---|---|---|---|
| ¹ Stenol® 1618L (ex. Cognis) | | | | |

The formulations are stored at 45°C and the viscosity measured periodically. The results are reported in Table 13. The figures in the table represent viscosity measurements (in units of mPa.s) as measured on a Haake RS600 Viscometer at a shear rate of 106 s⁻¹.

**Table 13**

| | 0 weeks | 2 weeks | 4 weeks | 7 weeks | 8 weeks | 10 weeks |
|---|---|---|---|---|---|---|
| Example I | 56 | 68 | 55 | 95 | 267 | Gel |
| Example J | 58 | 65 | 59 | 68 | 171 | Gel |
| Example K | 54 | 64 | 63 | 57 | 126 | 422 |
| Formulation 1 | 45 | 63 | 68 | 57 | 60 | 180 |

The results demonstrate that biasing the esterquat distribution towards TEQ and reducing esteramine content increases viscostability. Formulation 1 in accordance with the invention demonstrates improved stability.

The compositional data of the formulation after 8 weeks' storage at 45°C is reported in Table 14.

**Table 14**

| | Sample I | Sample J | Sample K | Formulation 1 |
|---|---|---|---|---|
| MEQ/wt.% | 1.43 | 1.40 | 1.48 | 1.36 |
| DEQ/wt.% | 1.00 | 1.02 | 1.25 | 1.46 |
| TEQ/wt.% | 0.11 | 0.11 | 0.15 | 0.21 |
| Free fatty acid/wt.% | 1.41 | 1.32 | 1.11 | 0.93 |
| Total esterquat/wt.%. | 2.54 | 2.53 | 2.88 | 3.03 |
| Wt.% fatty acid of total solids | 35.8 | 34.3 | 27.8 | 23.5 |

The results demonstrate that biasing the esterquat distribution towards TEQ and reducing esteramine content reduces hydrolysis rates.

### Example 2 and Examples L and M (Comparative)

Additional formulations are prepared as in Examples B and C but with esterquat raw materials having different esterquat distributions. The raw material compositional data used in the formulation is reported in Table 15.

**Table 15**

| | Example 2 | Example L | Examples M |
|---|---|---|---|
| MEQ/wt.% | 10.6 | 13.7 | 17.8 |
| DEQ/wt.% | 44.1 | 53.6 | 55.8 |
| TEQ/wt.% | 45.3 | 32.7 | 26.4 |
| Esteramine/wt.% | 5.34 | 6.49 | 7.18 |
| Free fatty acid/wt.% | 0.84 | 0.76 | 0.68 |

The formulations are stored at 45°C and the viscosity measured periodically. The results are reported in Table 16. The figures in the table represent viscosity measurements (in units of mPa.s) as measured on a Haake RS600 Viscometer at a shear rate of 106 s⁻¹.

**Table 16**

| | 0 weeks | 2 weeks | 4 weeks | 6 weeks | 8 weeks |
|---|---|---|---|---|---|
| Example 2 | 25 | 34 | 38 | 48 | 105 |
| Example L | 35 | 43 | 50 | 91 | 312 |
| Example M | 68 | 68 | 85 | 56 | 418 |

The results demonstrate that Example 2 in accordance with the invention having a high TEQ and low esteramine exhibits improved viscostability.

The formulation compositional data for the samples after 8 weeks storage at 45°C is reported in Table 17.

**Table 17**

| | Formulation 2 | Sample L | Sample M |
|---|---|---|---|
| MEQ/wt.% | 1.79 | 1.63 | 1.86 |
| DEQ/wt.% | 1.55 | 0.98 | 1.09 |
| TEQ/wt.% | 0.16 | 0.09 | 0.09 |
| Free fatty acid/wt.% | 1.57 | 1.59 | 1.73 |
| Total esterquat/wt.% | 3.5 | 2.7 | 3.04 |
| Wt.% fatty acid of total solids | 31.0 | 37.1 | 36.3 |

### Examples 3 & 4 and Example N & O (Comparative)

Additional formulations are prepared as in Examples B and C but with esterquat raw materials having different esterquat distributions. The raw material compositional data used in the formulation is reported in Table 18.

**Table 18**

| | Example 3 | Example 4 | Example N | Example O |
|---|---|---|---|---|
| OMEQ/wt.% | 19.8 | 18.9 | 18.7 | 19.7 |
| DEQ/wt.% | 54.7 | 54.3 | 56.4 | 57.8 |
| TEQ/wt.% | 25.5 | 26.9 | 24.9 | 22.5 |
| Esteramine/wt.% | 0.0 | 0.0 | 0.0 | 0.0 |
| Free fatty acid/wt.% | 0.30 | 0.41 | 0.33 | 0.57 |

The formulations are stored at 45°C and the viscosity measured periodically. The results are reported in Table 19. The figures in the table represent viscosity measurements (in units of mPa.s) as measured on a Haake RS600 Viscometer at a shear rate of 106 s⁻¹ (inverse seconds).

**Table 19**

| | 0 wks | 2 wks | 4 wks | 6 wks | 8 wks | 10 wks | 12 wks |
|---|---|---|---|---|---|---|---|
| Example 3 | 29 | 36 | 30 | 32 | 24 | 54 | 352 |
| Example 4 | 29 | 37 | 35 | 34 | 23 | 64 | 249 |
| Example N | 30 | 32 | 34 | 33 | 21 | 130 | 375 |
| Example O | 77 | 89 | 87 | 68 | 58 | 190 | 503 |

The results demonstrate that Examples 3 and 4 in accordance with the invention having a high TEQ and low esteramine exhibit improved viscostability.

The formulation compositional data for the samples after 8 weeks storage at 45°C is reported in Table 20. The analytical data supports the fact that the higher TEQ examples of the invention have lower rates of hydrolysis.

**Table 20**

| | Example 3 | Example 4 | Example N | Example O |
|---|---|---|---|---|
| MEQ/wt.% | 1.44 | 1.42 | 1.54 | 1.71 |
| DEQ/wt.% | 1.92 | 1.92 | 1.93 | 1.87 |
| TEQ/wt.% | 0.34 | 0.34 | 0.28 | 0.24 |
| Free fatty acid/wt.% | 0.68 | 0.67 | 0.77 | 0.89 |
| Total esterquat/wt.% | 3.69 | 3.67 | 3.75 | 3.82 |
| Wt.% fatty acid of total solids | 15.56 | 15.44 | 17.03 | 18.90 |

## Claims

1. A fabric conditioner active composition comprising an esterquat mixture of quaternised mono-, di-, and tri-esters of alkanolamines in which the tri-esterquat content of said esterquat mixture is from 25 to 50% by weight of said mixture, wherein:
(i) when the tri-esterquat content is from 25 to 30% by weight of said esterquat mixture, the free amine content of the composition is less than 0.5% by weight of said esterquat mixture,
(ii) when the tri-esterquat content is more than 30% by weight of said esterquat mixture, the free amine content of the composition is less than 6% by weight of said esterquat mixture, and
(iii) the free fatty acid content of the composition is less than 1% by weight of said esterquat mixture.

2. A composition as claimed in claim 1, wherein the quaternised mono-, di-, and tri-esters of alkanolamines are represented by formula (I): 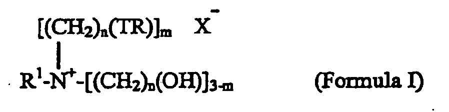 wherein each R is independently selected from a C₅₋₃₅ alkyl or alkenyl group, R¹ represents a C₁₋₄ alkyl or hydroxyalkyl group or a C₂₄ alkenyl group,
T is 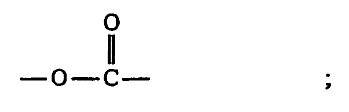 n is an integer selected from 1 to 4, m is 1 for an esterquat monoester, 2 for an esterquat diester, 3 for an esterquat triester, and denotes the number of moieties to which it refers that pend directly from the N atom, and X is an anionic group.

3. The composition of claim 2, wherein:
n is 2,3, or 4.

4. A composition as claimed in any preceding claim in which the quaternised monk-, di-, and tri-esters of alkanolamines are tallow fatty acid based triethanolamine ammonium compounds.

5. A composition as claimed in any preceding claim in which the ta-esterquat content is more than 30% and the free amine content is less than 5% by weight of the esterquat mixture.

6. A composition as claimed in any preceding claim in which each R is independently selected from a tallow alkyl or alkenyl group.

7. A composition as claimed in any preceding claim in which at least substantially each R is a hydrogenated tallow alkyl group.

8. A composition as claimed in claim 2, 3, 4, 5, 6, or 7, further **characterized** as the reaction product of a trialkanolamine having the formula:
N-[(CH₂)ₙ(OH)]₃,
a fatty acid having the formula:
H-T-R,
and a quaternising agent.

9. A composition as claimed in claim 8 in which the trialkanolamine is triethanolamine.

10. A composition as claimed in claim 8 or 9 in which the fatty acid is tallow fatty acid or hydrogenated tallow fatty acid.

11. A composition as claimed in any of claims 8, 9 or 10, in which the fatty acid has an iodine number of 0-20.

12. A composition as claimed in any preceding claim 8-11 in which the quaternising agent is R₁-X.

13. A composition as claimed in any preceding claim 8-12 which the quaternising agent is at least one C₁-C₄ halide, at least one C₁-C₄ alkyl sulphate, or a combination thereof.

14. A composition as claimed in any preceding claim 8-13 in which the quaternising agent is methyl chloride, dimethyl sulphate, diethyl sulphate, or a combination thereof.

## Patentansprüche

1. Weichspüler-wirksame Zusammensetzung, die ein Esterquat-Gemisch von quaternisierten Mono-, Di- und Tri-Estern von Alkanolaminen umfasst, wobei der Tri-Esterquat-Gehalt des Esterquat-Gemisches 25-50 Gew.-% des Gemisches beträgt, und wobei:
(i) der Gehalt an freiem Amin der Zusammensetzung weniger als 0,5 Gew.-% des Esterquat-Gemisches beträgt, wenn der Tri-Esterquat-Gehalt 25-30 Gew.-% des Esterquat-Gemisches beträgt,
(ii) der Gehalt an freiem Amin der Zusammensetzung weniger als 6 Gew.-% des Esterquat-Gemisches beträgt, wenn der Tri-Esterquat-Gehalt mehr als 30 Gew.-% des Esterquat-Gemisches beträgt, und
(iii) der Gehalt an freier Fettsäure der Zusammensetzung weniger als 1 Gel.-% des Esterquat-Gemisches beträgt.

2. Zusammensetzung gemäß Anspruch 1, wobei die quaternisierten Mono-, Di- und Tri-Ester von Alkanolaminen der Formel (I) entsprechen: 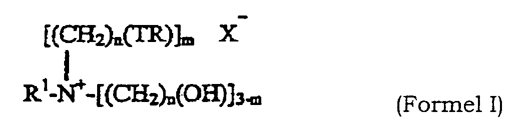 worin jede Gruppe R unabhängig ausgewählt ist aus einer C₅₋₃₅-Alkyl- oder Alkenylgruppe, R¹ eine C₁₋₄-Alkyl- oder Hydroxyalkylgruppe oder eine C₂₋₄-Alkenylgruppe darstellt,
T 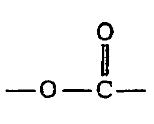 ist,
n eine ganze Zahl von 1 bis 4 ist, m 1 im Fall eines Esterquat-MonoEsters, 2 im Fall eines Esterquat-Di-Esters, 3 im Fall eines Esterquat-Tri-Esters beträgt und die Anzahl an Gruppen, auf die es sich bezieht, bezeichnet, die direkt an dem N-Atom hängen, und X- eine anionische Gruppe ist.

3. Zusammensetzung gemäß Anspruch 2, wobei n 2, 3 oder 4 beträgt.

4. Zusammensetzung gemäß einem der vorhergehenden Ansprüche, wobei die quaternisierten Mono-, Di- und Tri-Ester von Alkanolaminen Talgfettsäure-basierte Triethanolaminammoniumverbindungen sind.

5. Zusammensetzung gemäß einem der vorhergehenden Ansprüche, wobei der Tri-Esterquat-Gehalt mehr als 30% beträgt und der Gehalt an freiem Amin weniger als 5 Gew.-% des Esterquat-Gemisches beträgt.

6. Zusammensetzung gemäß einem der vorhergehenden Ansprüche, wobei jede Gruppe R unabhängig ausgewählt ist aus einer Talgalkyl- oder Alkenylgruppe.

7. Zusammensetzung gemäß einem der vorhergehenden Ansprüche, wobei zumindest im Wesentlichen jede Gruppe R eine hydrierte Talgalkylgruppe ist.

8. Zusammensetzung gemäß Anspruch 2, 3, 4, 5, 6 oder 7, die ferner als das Reaktionsprodukt eines Trialkanolamins der Formel:
N-[(CH₂)ₙ(OH)]₃
einer Fettsäure der Formel:
H-T-R
und eines Quaternisierungsmittels charakterisiert ist.

9. Zusammensetzung gemäß Anspruch 8, wobei das Trialkanolamin Triethanolamin ist.

10. Zusammensetzung gemäß Anspruch 8 oder 9, wobei die Fettsäure Talgfettsäure oder hydrierte Talgfettsäure ist.

11. Zusammensetzung gemäß einem der Ansprüche 8, 9 oder 10, wobei die Fettsäure eine Iodzahl von 0-20 aufweist.

12. Zusammensetzung gemäß einem der vorhergehenden Ansprüche 8-11, wobei das Quaternisierungsmittel R₁-X ist.

13. Zusammensetzung gemäß einem der vorhergehenden Ansprüche 8-12, wobei das Quaternisierungsmittel mindestens ein C₁-C₄-Halogenid, mindestens ein C₁-C₄-Alkylsulfat oder eine Kombination davon ist.

14. Zusammensetzung gemäß einem der vorhergehenden Ansprüche 8-13, wobei das Quaternisierungsmittel Methylchlorid, Dimethylsulfat, Diethylsulfat oder eine Kombination davon ist.

## Revendications

1. Composition active pour le conditionnement de tissus, comprenant un mélange d'esters quaternaires composés de mono, di-, et triesters d'alcanolamines quaternisés, dans laquelle la teneur en tri-ester quaternaire du dit mélange d'esters quaternaires représente de 25 à . 50% en poids du dit mélange, tandis que:
(i) lorsque la teneur en tri-ester quaternaire est de 25 à 30% en poids du dit mélange d'esters quaternaires, la teneur en amine libre de la composition est inférieure à 0,5% en poids du dit mélange d'esters quaternaires,
(ii) lorsque la teneur en tri-ester quaternaire est supérieure à 30% en poids du dit mélange d'esters quaternaires, la teneur en amine libre de la composition est inférieure à 6% en poids du dit mélange d'esters quaternaires, et
(iii) la teneur en acide gras libre de la composition est inférieure à 1% en poids du dit mélange d'esters quaternaires.

2. Composition selon la revendication 1, dans laquelle les mono-, di-, et tri-esters d'alcanolamines quaternisés sont représentés par la formule (I): 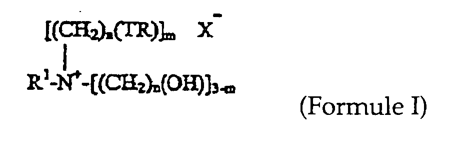 où chaque R est indépendamment choisi parmi un groupe alkyle ou alcényle en C₅-₃₅, R¹ représente un groupe alkyle ou hydroxyalkyle en C₁₋₄ ou un groupe alcényle en C₂₋₄,
T est 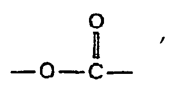 n est un entier choisi de 1 à 4, m est 1 pour un ester quaternaire monoester, 2 pour un ester quaternaire diester, 3 pour un ester quaternaire triester, et désigne le nombre de portions auxquelles ils se réfèrent qui pendent directement de l'atome N, et X- est un groupe anionique.

3. Composition selon la revendication 2, dans laquelle:
n est 2, 3, ou 4.

4. Composition selon l'une quelconque des revendications précédentes, dans laquelle les mono-, di-, et tri-esters d'alcanolamines quaternisés sont des composés de triéthanolamine ammonium à base d'acide gras de suif.

5. Composition selon l'une quelconque des revendications précédentes, dans laquelle la teneur en tri-ester quaternaire est supérieure à 30% et la teneur en amine libre est inférieure à 5% en poids du mélange d'esters quaternaires.

6. Composition selon l'une quelconque des revendications précédentes, dans laquelle chaque R est indépendamment choisi parmi un groupe alkyle ou alcényle de suif.

7. Composition selon l'une quelconque des revendications précédentes, dans laquelle au moins substantiellement chaque R est un groupe alkyle de suif hydrogéné.

8. Composition selon les revendications 2, 3, 4, 5, 6, ou 7, **caractérisée en** outre comme étant le produit de réaction d'une trialcanolamine ayant la formule:
N-[(CH₂)n(OH)]₃,
un acide gras ayant la formule:
H-T-R,
et un agent de quaternisation.

9. Composition selon la revendication 8, dans laquelle la trialcanolamine est la triéthanolamine.

10. Composition selon la revendication 8 ou 9, dans laquelle l'acide gras est l'acide gras de suif, ou l'acide gras de suif hydrogéné.

11. Composition selon l'une quelconque des revendications 8, 9, ou 10, dans laquelle l'acide gras a un indice d'iode de 0-20.

12. Composition selon l'une quelconque des revendications précédentes 8-11, dans laquelle l'agent de quaternisation est R₁-X.

13. Composition selon l'une quelconque des revendications précédentes 8-12, dans laquelle l'agent de quaternisation est au moins un halogénure en C₁-C₄, au moins un alkyl(C₁-C₄)sulfate, ou une combinaison de ceux-ci.

14. Composition selon l'une quelconque des revendications précédentes 8-13, dans laquelle l'agent de quaternisation est le chlorure de méthyle, le sulfate de diméthyle, le sulfate de diéthyle, ou une combinaison de ceux-ci.
